# EUROPÄISCHE PATENTSCHRIFT

(11) **EP 0 790 251 B1**
(45) Veröffentlichungstag und Bekanntmachung des Hinweises auf die Patenterteilung: **04.12.2002**
(21) Anmeldenummer: 97101270.3
(22) Anmeldetag: 28.01.1997
(51) Int. Cl.: C07F 3/02, C07C 209/66

(54) **Herstellung und Verwendung von (3-Alkoxyphenyl)magnesiumchloriden**
Preparation and use of (3-alkoxyphenyl) magnesium chlorides
Préparation et utilisation de (3-alkoxyphényl) magnésiumchlorures

(30) Priorität: 16.02.1996 DE 19605778
(43) Veröffentlichungstag der Anmeldung: 20.08.1997
(73) Patentinhaber: Grünenthal GmbH, 52078 Aachen (DE)
(72) Erfinder: Finkam, Michael, Dr., 52066 Aachen (DE); Kohnen, Thomas, 52223 Stolberg (DE); Winter, Werner, Prof. Dr., 52078 Aachem (DE)

(56) Entgegenhaltungen:
- FLICK K ET AL: "UNTERSUCHUNGEN ZUR CHEMISCHEN STRUKTUR UND ANALGETISCHEN WIRKUNG VON PHENYLSUBSTITUIERTEN AMINOMETHYLCYCLOHEXANOLEN. STUDIES ON CHEMICAL STRUCTURE AND ANALGETIC ACTIVITY OF PHENYL SUBSTITUTED AMINOMETHYLCYCLOHEXANOLES" ARZNEIMITTEL FORSCHUNG. DRUG RESEARCH, Bd. 28, Nr. 1A, 1. Januar 1978 (1978-01-01), Seiten 107-113, XP000608150 ISSN: 0004-4172

## Beschreibung

Die Erfindung betrifft ein Verfahren zur Herstellung von (3-Alkoxyphenyl)magnesiumchloriden, sowie die Verwendung dieser Verbindungen.

Zahlreiche pharmazeutische und agrochemische Wirkstoffe enthalten die m-Anisylgruppe. Diese wird vorzugsweise mittels einer metallorganischen m-Anisyl-Verbindung, insbesondere mittels einer m-Anisylgrignard-Verbindung in die zu synthetisierende Verbindung eingeführt. Als Grignard-Verbindung wird hierbei m-Anisylmagnesiumbromid eingesetzt, das durch Umsetzung von m-Anisylbromid mit metallischem Magnesium in einem Lösungsmittel erhalten wird (Arzn. Forsch./Drug Res. 28 (I), 107 (1978)).

Üblicherweise werden bei der Herstellung von Grignard-Verbindungen die reaktiven Bromide eingesetzt. Aus ökonomischer und ökologischer Sicht hat jedoch die Substitution der Bromide durch die weniger reaktiven Chloride bei der Herstellung von Grignard-Reagenzien Vorteile, da die Chloride zum einen kostengünstiger sind und zum anderen bedingt durch ihr niedrigeres Molekulargewicht geringere Salzmengen verursachen. Darüber hinaus werden bei Verwendung der Chloride weniger Nebenprodukte gebildet.

Aus US-A-2 959 596 und J. Chem. Soc. 1968, 1265 ist die Herstellung und Verwendung von o- und p-Anisylmagnesiumchlorid bekannt. In JP-A-60/72833, referiert in Derwent WPI Acc No: 85-137805/23, wird (4-Ethoxyphenyl)magnesiumchlorid ohne Angabe des Herstellungsverfahrens offenbart. Die Herstellung und Verwendung von (3-Methoxyphenyl)- und (3-Ethoxyphenyl)magnesiumchlorid ist dagegen bisher nicht beschrieben worden. Dies läßt sich mit der elektronischen Desaktivierung des Aromaten durch die Alkoxygruppe in der meta-Position erklären.

Die der Erfindung zugrundeliegende Aufgabe bestand daher in der Entwicklung eines Verfahrens zur Herstellung von (3-Alkoxyphenyl)magnesiumchloriden, in den der Alkoxyrest Methoxy, Ethoxy, Propoxy, Isopropoxy, n-Butoxy oder Cyclopentoxy bedeutet.

Überraschenderweise wurde gefunden, daß sich (3-Alkoxyphenyl)magnesiumchloride mit ein bis fünf Kohlenstoffatomen im Alkoxyrest, in dem der Alkoxyrest Methoxy, Ethoxy, Propoxy, Isopropoxy, n-Butoxy oder Cyclopentoxy bedeutet, durch Umsetzung der entsprechenden 3-Alkoxyphenylchloride mit aktiviertem Magnesium in hohen Ausbeuten erhalten lassen.

Erfindungsgegenstand ist dementsprechend ein Verfahren zur Herstellung von (3-Alkoxyphenyl)magnesiumchloriden mit ein bis fünf Kohlenstoffatomen im Alkoxyrest, in dem der Alkoxyrest Methoxy, Ethoxy, Propoxy, Isopropoxy, n-Butoxy oder Cyclopentoxy bedeutet, welche durch Umsetzung eines 3-Alkoxyphenylchlorids mit aktiviertem Magnesium, erhalten durch Reduktion von Magnesiumhalogeniden mit einem Alkalimetall, hergestellt werden.

Für das erfindungsgemäße Verfahren eignet sich ihsbesondere aktiviertes Magnesium, das durch Produktion von Magnesiumhalogeniden, insbesondere Magnesiumchlorid mit Lithium, Natrium oder Kalium erhalten wird (J. Org. Chem. 52, 3674 (1987); J. Am. Chem. Soc. 96, 1775 (1974)). Die Reduktion wird üblicherweise mit einem 1 bis 5 %igen molaren Überschuß an Magnesiumhalogenid in einem Lösungsmittel oder Lösungsmittelgemisch, beispielsweise aliphatischen Ethern, wie Tetrahydrofuran, substituierten Tetrahydrofuranen, Dimethoxyethan und/oder Dimethyldiglycol, bei Temperaturen zwischen 65° C und 162° C durchgeführt. Es kann vorteilhaft sein, die Reduktion in Gegenwart von Alkali- und Erdalkalisalzen, beispielsweise Alkalijodiden, Alkalisulfaten und/oder Erdalkalisulfaten durchzuführen. Das durch Reduktion erhaltene aktivierte Magnesium wird vorzugsweise ohne Isolierung mit einem 3-Alkoxyphenylchlorid mit ein bis fünf Kohlenstoffatomen im Alkoxyrest, in dem der Alkoxyrest Methoxy, Ethoxy, Propoxy, Isopropoxy, n-Butoxy oder Cyclopentoxy bedeutet, zu der entsprechenden Grignard-Verbindung umgesetzt. Besonders geeignet zur Umsetzung mit aktiviertem Magnesium sind 3-Methoxyphenylchlorid und 3-Ethoxyphenylchlorid.

Nach dem erfindungsgemäßen Verfahren lassen sich die Magnesiumchlorid-Verbindungen ohne Bildung von Nebenprodukten in hohen Ausbeuten herstellen. Werden dagegen 3-Alkoxyphenylchloride mit nicht aktiviertem Magnesium in Gegenwart geringer Mengen von Dibromethan oder nach dem in EP-A-307 106 für 3-t-Butoxyphenylchlorid beschriebenen Verfahren mit nicht aktiviertem Magnesium in Gegenwart von Ethylbromid umgesetzt, werden die entsprechenden Grignard-Verbindungen nur in unbefriedigenden Ausbeuten erhalten. Darüber hinaus kommt es in erheblichem Umfang zur Bildung von Nebenprodukten.

Mit den erfindungsgemäß hergestellten (3-Alkoxyphenyl)-magnesiumchloriden lassen sich Aldehyde und Ketone in guten Ausbeuten zu den entsprechenden Alkoholen umsetzen.

Weiterer Erfindungsgegenstand ist daher die Verwendung eines (3-Alkoxyphenyl)magnesiumchlorids mit ein bis fünf Kohlenstoffatomen im Alkoxyrest, in dem der Alkoxyrest Methoxy, Ethoxy, Propoxy, Isopropoxy, n-Butoxy oder Cyclopentoxy bedeutet, zur Umsetzung mit einem β-Aminoaldehyd oder β-Aminoketon der Formel I 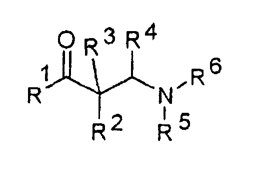 in der R¹ H oder C₁₋₄-Alkyl darstellt, R² H oder C₁₋₄-Alkyl oder R² zusammen mit R¹ -(CH₂)₄- oder R² zusammen mit R³ C₄₋₇-Cycloalkyl oder R² zusammen mit R⁴ C₅₋₃-Cycloalkyl oder R² zusammen mit R⁵ einen fünf- bis achtgliedrigen Heterocyclus bedeutet, R³ H oder geradkettiges C₁₋₄-Alkyl darstellt, R⁴ H ist und R⁵ C₁₋₃-Alkyl und R⁶ C₁₋₃-Alkyl bedeuten, oder zur Umsetzung mit einem Aldehyd oder Keton der Formel II 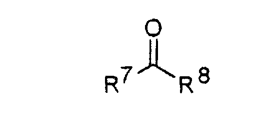 in der R⁷ und R⁸ gleich oder verschieden sind und jeweils H, C₁₋₆-Alkyl oder C₃₋₆-Cycloalkyl bedeuten.

Besonders bevorzugt wird (3-Methoxyphenyl)magnesiumchlorid oder (3-Ethoxyphenyl)magnesiumchlorid zur Umsetzung mit einem β-Aminoaldehyd oder β-Aminoketon verwendet. Besonders geeignete β-Aminoaldehyde oder β-Aminoketone sind solche der Formel I, in der R¹ C₁₋₄-Alkyl ist, R² H oder C₁₋₄-Alkyl oder R² zusammen mit R¹ -(CH₂)₄- bedeutet, R³ H oder geradkettiges C₁₋₄-Alkyl bedeutet und R⁴ H, R⁵ CH₃ und R⁶ CH₃ darstellen.

Die Umsetzung einer erfindungsgemäß hergestellten Magnesiumchloridverbindung wird in an sich bekannter Weise durchgeführt, indem die Grignard-Verbindung in einem Lösungsmittel oder einem Lösungsmittelgemisch, beispielsweise aliphatischen Ethern, wie Tetrahydrofuran, substituierten Tetrahydrofuranen, Dialkylethern und/oder Dioxan, und/oder aromatischen Kohlenwasserstoffen, wie Benzol, Toluol und/oder Xylol, bei Temperaturen zwischen -78° C und 120° C mit einer Verbindung der Formel I oder II zur Reaktion gebracht wird.

### Beispiele

Alle Arbeiten wurden mit getrockneten Lösungsmitteln und Reagenzien unter Schutzgasatmosphäre durchgeführt.

### Beispiel 1

### Herstellung von (3-Methoxyphenyl)magnesiumchlorid

2,04 g (21,4 mmol) Magnesiumchlorid wurden in einen 100 ml Dreihalskolben, ausgestattet mit Rückflußkühler, Thermometer und Tropftrichter, gegeben und mit 50 ml Tetrahydrofuran (THF) überschichtet. Es wurden 0,82 g (21,0 mmol) frisch geschnittenes Kalium portionsweise hinzugegeben und unter Rühren 90 Minuten unter Rückfluß erhitzt. Danach wurden innerhalb von 30 Minuten 3,05 g (21,4 mmol) 3-Chloranisol, gelöst in 20 ml THF, unter Rückfluß zugetropft. Nach beendeter Zugabe wurde 20 Stunden bei Raumtemperatur gerührt und die erhaltene Grignard-Verbindung zur weiteren Umsetzung eingesetzt.

### Beispiel 2

### Herstellung von 2-((Dimethylamino)methyl)-1-(3-methoxyphenyl)-cyclohexanol

Zur Lösung des nach Beispiel 1 erhaltenen (3-Methoxyphenyl)magnesiumchlorids wurden unter Eisbadkühlung 3,32 g (21,4 mmol) 2- (Dimethylamino)methyl-cyclohexanon, gelöst in 10 ml THF, innerhalb von 40 Minuten getropft. Nach 24-stündigem Rühren bei Raumtemperatur wurde unter Eisbadkühlung mit 20 ml 20%iger Ammoniumchloridlösung hydrolisiert. Die organische Phase wurde abgetrennt und die wäßrige Phase noch zweimal mit Essigsäureethylester extrahiert. Die vereinigten organischen Phasen wurden über Magnesiumsulfat getrocknet und filtriert. Nach destillativer Entfernung des Lösungsmittels wurden 3,4 g (60 der Theorie) der Cyclohexanolverbindung erhalten.

## Patentansprüche

1. Verfahren zur Herstellung von (3-Alkoxyphenyl)magnesiumchloriden mit ein bis fünf Kohlenstoffatomen im Alkoxyrest durch Umsetzung eines entsprechenden (3-Alkoxyphenyl)chlorids, in dem der Alkoxyrest Methoxy, Ethoxy, Propoxy, Isopropoxy, n-Butoxy oder Cyclopentoxy bedeutet, mit aktiviertem Magnesium, erhalten durch Reduktion von Magnesiumhalogeniden mit einem Alkalimetall.

2. Verfahren nach Anspruch 1 **dadurch gekennzeichnet, daß** aktiviertes Magnesium durch Reduktion von Magnesiumchlorid mit Lithium, Natrium oder Kalium erhalten wird.

3. Verfahren nach einem oder mehreren der Ansprüche 1 bis 2 **dadurch gekennzeichnet, daß** (3-Methoxyphenyl)chlorid oder (3-Ethoxyphenyl)chlorid mit aktiviertem Magnesium umgesetzt wird.

4. Verwendung eines (3-Alkoxyphenyl)magnesiumchlorids mit ein bis fünf Kohlenstoffatomen im Alkoxyrest, in dem der Alkoxyrest Methoxy, Ethoxy, Propoxy, Isopropoxy, n-Butoxy oder Cyclopentoxy bedeutet, zur Umsetzung mit einem β-Aminoaldehyd oder β-Aminoketon der Formel I 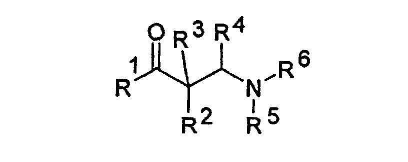 in der R¹ H oder C₁₋₄-Alkyl bedeutet, R² H oder C₁₋₄-Alkyl oder R² zusammen mit R¹ -(CH₂)₄- oder R² zusammen mit R³ C₄₋₇-Cycloalkyl oder R² zusammen mit R⁴ C₅₋₈-Cycloalkyl oder R² zusammen mit R⁵ einen fünf- bis achtgliedrigen Heterocyclus bedeutet, und R³ H oder geradkettiges C₁₋₄-Alkyl, R⁴ H, R⁵ C₁₋₃-Alkyl und R⁶ C₁₋₃-Alkyl darstellen, oder zur Umsetzung mit einem Aldehyd oder Keton der Formel II 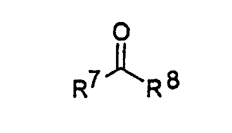 in der R⁷ und R⁸ gleich oder verschieden sind und jeweils H, C₁₋₆-Alkyl oder C₃₋₆-Cycloalkyl darstellen.

5. Verwendung nach Anspruch 4 **dadurch gekennzeichnet, daß** ein (3-Alkoxyphenyl)magnesiumchlorid, in dem der Alkoxyrest Methoxy, Ethoxy, Propoxy, Isopropoxy, n-Butoxy oder Cyclopentoxy bedeutet, zur Umsetzung mit einem β-Aminoaldehyd oder β-Aminoketon verwendet wird.

6. Verwendung nach einem oder beiden der Ansprüche 4 und 5 **dadurch gekennzeichnet, daß** ein (3-Alkoxyphenyl)magnesiumchlorid zur Umsetzung mit einem β-Aminoaldehyd oder einem β-Aminoketon der Formel I, in der R¹ C₁₋₄-Alkyl ist, R² H oder C₁₋₄-Alkyl oder R² zusammen mit R¹ -(CH₂)₄- darstellt, und R³ H oder geradkettiges C₁₋₄-Alkyl, R⁴ H, R⁵ CH₃ und R⁶ CH₃ darstellen, verwendet wird.

7. Verwendung nach einem oder mehreren der Ansprüche 4 bis 6 **dadurch gekennzeichnet, daß** (3-Methoxyphenyl)magnesiumchlorid oder (3-Ethoxyphenyl)magnesiumchlorid verwendet wird.

## Claims

1. Process for the production of (3-alkoxyphenyl)-magnesium chlorides with one to 5 carbon atoms in the alkoxy radical by reacting a corresponding (3-alkoxyphenyl) chloride, in which the alkoxy radical denotes methoxy, ethoxy, propoxy, isopropoxy, n-butoxy or cyclopentoxy, with activated magnesium obtained by reduction of magnesium halides with an alkali metal.

2. Process according to claim 1, **characterised in that** activated magnesium is obtained by reduction of magnesium chloride with lithium, sodium or potassium.

3. Process according to one or both of claims 1 and 2, **characterised in that** (3-methoxyphenyl) chloride or (3-ethoxyphenyl) chloride is reacted with activated magnesium.

4. Use of a (3-alkoxyphenyl)magnesium chloride with one to five carbon atoms in the alkoxy radical, in which the alkoxy radical denotes methoxy, ethoxy, propoxy, isopropoxy, n-butoxy or cyclopentoxy, for reaction with a β-aminoaldehyde or β-aminoketone of the formula I 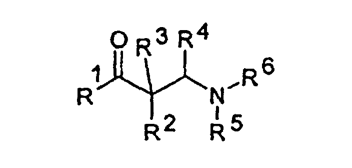 in which R¹ denotes H or C₁₋₄-alkyl, R² denotes H or C₁₋₄-alkyl or R² together with R¹ denote -(CH₂)₄- or R² together with R³ denote C₄₋₇-cycloalkyl or R² together with R⁴ denote C₅₋₈-cycloalkyl or R² together with R⁵ denote a five-membered to eight-membered heterocyclic ring, R³ denotes H or straight-chain C₁₋₄-alkyl, R⁴ is H, R⁵ denotes C₁₋₃-alkyl and R⁶ denotes C₁₋₃-alkyl, or for the reaction with an aldehyde or ketone of the formula II 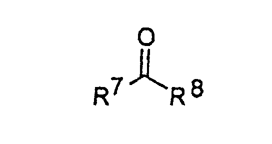 in which R⁷ and R⁹ are identical or different and in each case denote H, C₁₋₆-alkyl or C₃₋₆-cycloalkyl.

5. Use according to claim 4, **characterised in that** a (3-alkoxyphenyl)magnesium chloride in which the alkoxy radical denotes methoxy, ethoxy, propoxy, isopropoxy, n-butoxy or cyclopentoxy, is used for the reaction with a β-aminoaldehyde or β-aminoketone.

6. Use according to one or both of claims 4 and 5, **characterised in that** a (3-alkoxyphenyl)magnesium chloride is used for the reaction with a β-aminoaldehyde or a β-aminoketone of the formula I, in which R¹ is C₁₋₄-alkyl, R² is H or C₁₋₄-alkyl or R² together with R¹ denotes -(CH₂)₄-, and R³ denotes H or straight-chain C₁₋₄-alkyl, R⁴ denotes H, R⁵ denotes CH₃ and R⁶ denotes CH₃.

7. Use according to one or more of claims 4 to 6, **characterised in that** (3-methoxyphenyl)magnesium chloride or (3-ethoxyphenyl)magnesium chloride is used.

## Revendications

1. Procédé de production de chlorures de (3-alkoxyphényl)magnésium portant un reste alkoxy ayant 1 à 5 atomes de carbone par réaction d'un chlorure de 3-alkoxyphényle correspondant dans lequel le reste alkoxy désigne un reste méthoxy, éthoxy, propoxy, isopropoxy, n-butoxy ou cyclopentoxy, avec du magnésium activé obtenu par réduction d'halogénures de magnésium avec un métal alcalin.

2. Procédé suivant la revendication 1, **caractérisé en ce que** le magnésium activé est obtenu par réduction de chlorure de magnésium avec du lithium, du sodium ou du potassium.

3. Procédé suivant l'une des revendications 1 et 2 ou les deux, **caractérisé en ce qu'**on fait réagir du chlorure de (3-méthoxyphényle) ou du chlorure de (3-éthoxyphényle) avec du magnésium activé.

4. Utilisation d'un chlorure de (3-alkoxyphényl)-magnésium ayant 1 à 5 atomes de carbone dans le reste alkoxy, qui désigne un reste méthoxy, éthoxy, propoxy, isopropoxy, n-butoxy ou cyclopentoxy, pour la réaction avec un β-amino-aldéhyde ou une β-aminocétone de formule (I) 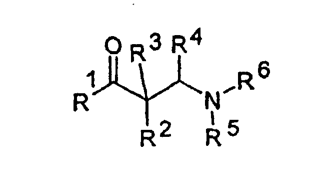 dans laquelle R¹ représente H ou un groupe alkyle en C₁ à C₄, R² représente H ou un groupe alkyle en C₁ à C₄, ou bien R² forme conjointement avec R¹ un groupe -(CH₂)₄-, ou bien R² forme conjointement avec R³ un groupe cycloalkyle en C₄ à C₇ ou bien R² forme conjointement avec R⁴ un groupe cycloalkyle en C₅ à C₈ ou bien R² forme conjointement avec R⁵ un hétérocycle pentagonal à octagonal, et R³ représente H ou un groupe alkyle linéaire en C₁ à C₄, R⁴ représente H, R⁵ représente un groupe alkyle en C₁ à C₃ et R⁶ représente un groupe alkyle en C₁ à C₃, ou pour la réaction avec un aldéhyde ou une cétone de formule II 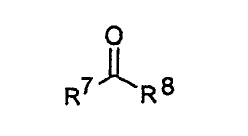 dans laquelle R⁷ et R⁸ sont identiques ou différents et représentent chacun H, un groupe alkyle en C₁ à C₆ ou cycloalkyle en C₃ à C₆.

5. Utilisation suivant la revendication 4, **caractérisée en ce qu'**on utilise un chlorure de (3-alkoxyphényl)magnésium dont le reste alkoxy désigne un reste méthoxy, éthoxy, propoxy, isopropoxy, n-butoxy ou cyclopentoxy, pour la réaction avec un β-amino-aldéhyde ou une β-aminocétone.

6. Utilisation suivant l'une des revendications 4 et 5 ou les deux, **caractérisée en ce qu'**on utilise un chlorure de (3-alkoxyphényl)magnésium pour la réaction avec un β-amino-aldéhyde ou une β-aminocétone de formule I dans laquelle R¹ est un groupe alkyle en C₁ à C₄, R² représente H ou un groupe alkyle en C₁ à C₄, ou bien R² forme conjointement avec R¹ un groupe -(CH₂)₄- et R³ représente H ou un groupé alkyle linéaire en C₁ à C₄, R⁴ représente H, R⁵ est un groupe CH₃ et R⁶ est un groupe CH₃.

7. Utilisation suivant une ou plusieurs des revendications 4 à 6, **caractérisée en ce qu'**on utilise le chlorure de (3-méthoxyphényl)magnésium ou le chlorure de (3-éthoxyphényl)magnésium.
